Europäisches Patentamt

**European Patent Office**

Office européen des brevets

(11) Publication number: **0 220 091**
A1

## (12) EUROPEAN PATENT APPLICATION

(21) Application number: **86402022.7**

(22) Date of filing: **16.09.86**

(51) Int. Cl.⁴: **A 61 F 13/20**
**B 31 C 1/00**

(30) Priority: **19.09.85 US 777974**

(43) Date of publication of application:
**29.04.87 Bulletin 87/18**

(84) Designated Contracting States:
**AT BE CH DE FR GB IT LI LU NL SE**

(71) Applicant: **KIMBERLY-CLARK CORPORATION**
**401 North Lake Street**
**Neenah Wisconsin 54956(US)**

(72) Inventor: **Tews, Richard Roy**
**1407 West Pershing Street**
**Appleton, Wisconsin(US)**

(74) Representative: **Sauvage, Renée**
**CABINET SAUVAGE 100 bis, avenue de Saint-Mandé**
**F-75012 Paris(FR)**

(54) Bias cut paper for convolutely wound tampon tubes.

(57) A convolutely wound paper tampon tube is described wherein a paper blank (A) having a narrow pointed leading edge (5) is fed to a mandrel (20) to be wound into a convolute tube. The blank may be in the form of a parallelogram, where the leading edge forms an angle with respect to one of the parallel sides of the blank or, the edge may be curved between two parallel sides of the blank. Alternatively, the edge may have a point formed by two sections intersecting at an angle. The trailing edge of the blank, in each instance, has an identical pattern to the leading edge.

FIG. 6

EP 0 220 091 A1

# BIAS CUT PAPER FOR CONVOLUTELY WOUND TAMPON TUBES

## TECHNICAL FIELD

The present invention relates, generally, to the field of catamenial tampons for personal feminine care or protection in order to absorb or otherwise contain menstrual fluids or similar exudate. The present invention relates more particularly to a paper tube particularly useful for the insertion of a tampon and a method for making the same. Of special concern is a paper tube that is readily disposable in cold water and can be flushed down the toilet.

## DESCRIPTION OF THE BACKGROUND ART

All manner and variety of devices or appliances configured for the absorption of such body fluids as menses are of course well known. As a class, these articles ought to possess certain necessary attributes of absorbency, comfort and psychological as well as physiological or physical protection. Desirably, these devices also are characterized as being discreet both during wear and when carried upon the wearer's person in anticipation of use. As will be seen from the ensuing discussion, these objectives are somewhat antagonistic in the sense that attainment of one has heretofore ordinarily necessitated compromising one or more other desirable feature.

The art has offered two basic types of feminine protection device with those objectives borne in mind; sanitary napkins or pads have been developed for external wear about the vulvar region of a user while tampons have been developed for residence within the

2

vaginal cavity and interruption of menstrual flow therefrom. Each offers distinct advantages and, as one would expect, distinct or peculiar disadvantages, which range from psychological impediments and/or concerns to physical discomfiture.

Looking to tampons as such an alternative to sanitary napkins, the same are preferred by a substantial number of women. Offering the ability to intercept menses within the vaginal canal, the disadvantages inhering in sanitary napkins in respect of covers and attachment means are overcome by this approach. That is not to say, however, that tampons afford a superior means of feminine protection for all users. For example, there are many individuals who, for either physical or psychological reasons, are unable to utilize tampons as an effective means of feminine protection. Tampons themselves may be found lacking in terms of construction inasmuch as efficacy relies significantly on the ability of the same to undergo radial expansion upon fluid swelling in order to form a seal or zone of occlusion within the vaginal canal. Failure to do so implies overall failure of the tampon to serve as a reliable protection device.

While there are essentially three types of tampons, plunger, stick and digital, the most preferable type of tampon in the United States is the plunger type. The tubes for the plunger type are either of the disposable or non-disposable variety. During the past several decades, increasing emphasis has been placed upon the ease of disposability of consumer goods after the goods have performed their intended purpose. A paper tampon tube, to be easily

3

disposable must be formed with an adhesive readily soluble in cold water and preferably constructed so that delamination of the tube occurs in a short period of time.

Plunger-type tampons are generally formed in two parts with an outer tube having an inner diameter slightly greater than the outer diameter of the tampon pledget inserted therein. The second part of a tampon inserter means is some type of plunger which operates in cooperation with the tube to expel the pledget. The tubes are generally made of paper products such as cardboard or thermoplastic. Further, thermoplastic tubes generally have an insertion end which forms a hemispherical profile around the leading edge of the tampon to protect it and maintain its integrity during insertion. Commercially available tampons utilizing cardboard tubes, however, generally do not have this type of closure, but rather the leading edge of the tampon extends beyond the tube end. Both thermoplastic and paper derived tubes can be made with a reduced diameter base which can be used for gripping or to better maintain the plunger used for expulsion, or for both purposes.

The ideal tampon tube should be inexpensive, simple to make, easily disposable, attractive and hygienic. Both molded plastic and paper inserter tubes have not been completely satisfactory in meeting these desirable attributes. There are no commercially available tubes molded from thermoplastics that are water disposable or flushable. Furthermore, the relative cost of thermoplastic is substantially greater than tubes which are made from paper or paper

4

products. In addition, thermoplastic molded tubes having a hemispherical shaped leading edge comprising individual arcuate shaped lobes are extremely difficult to mold without providing lobes having sharp edges or flashing, i.e. irregularly shaped burrs of plastic. Such tubes could provide problems when being withdrawn from the vagina. Paper tubes, on the other hand, also have problems. Commercially available cardboard tubes for tampons are generally spirally wound and open at the leading edge which may result in a fiber slough when the tampon is inserted. Such tubes generally have a relatively low beam strength, that is, a low resistance to a radial collapsing of the tube. One example of such spirally wound paper tubes can be found in U.S. Patent No. 3,764,438, issued October 9, 1973. After the outer tube is formed, the end through which the tampon is ejected may be shaped to have a tapered portion of smaller diameter than the diameter of the tampon. Such tapered portion has a generally round termination with corrugations on slits to form flexible petal portions through which the tampon can be forcibly ejected by the inner telescoping tube. The adhesive for holding the spiral layers together may be water soluble so that the paper tubes may be thrown into a toilet and flushed into the drain pipe. The water soluble adhesive causes delamination of the spiral layers so that the rigid tube structure is quickly softened. The paper is of a type chosen to disintegrate in water.

5

Another method of making paper tubes is to wind a rectangular sheet of paper on a mandrel to form a convoluted tube. Such tubes pose manufacturing problems because the feeding and alignment of the leading edge of the rectangular sheet of paper to a winding mandrel is difficult and care must be taken to insure precise alignment of the entire leading edge when fed to a winding mandrel.

There exists a need in the art for paper tube that is easy to manufacture, that has a high beam strength, and that is easily disposable.

The present invention solves such need, as set forth hereinafter in the description of exemplary embodiments.

OBJECTS AND SUMMARY OF THE INVENTION

An object of the invention is the manufacture of a convolute tube having a relatively high beam strength.

Another object of the invention is the manufacture of a convolute tube whereby feeding of the leading edge of a paper blank to a winding apparatus is facilitated.

A further object of the invention is a paper tube having additional edge areas to facilitate delamination of the tube when thrown in water.

Another object of the invention is the manufacture of a convolute tube using a water dispersible heat activatable adhesive for holding the coils of the tube together.

These and other objects are accomplished by the provision of a paper blank having a narrow pointed

6

leading edge to be fed to a winding mechanism for forming a tube. The narrow pointed leading edge can be in any shape such as, for example, the corner of a paper blank in the shape of a parallelogram where preferably the leading edge presents an angle between 5 degrees and 85 degrees. Rather than a straight leading edge of the parallelogram, a curved edge may be used. Alternatively, the leading edge may be pointed like an arrowhead. In each instance, the pattern of the leading edge is duplicated on the trailing edge. When formed into a tube, the leading edge of the paper blank gives the appearance of a spiral winding on the inside of the tube and the trailing edge gives the appearance of a spiral winding on the outside of the tube. Because the leading and trailing edges of the paper blanks of the invention are substantially longer than edges of a rectangular blank, the tube formed from the blank of the invention has substantially more edge area to cause the tube to delaminate faster when making water soluble tampon tubes. The narrow pointed leading edge further facilitates the feeding and alignment of the paper blank to a winding apparatus.

These and other objects and advantages will become more apparent from a description of the exemplary embodiments of the invention.

BRIEF DESCRIPTION OF THE DRAWINGS

Figs. 1-3 show front elevational views of paper blanks to be used in the manufacture of tampon tubes;

Fig. 4 schematically shows a paper blank being fed to a rotating mandrel;

7

Fig. 5 schematically shows a paper blank with its leading end being wound by a rotating mandrel; and

Fig. 6 is a perspective view of a tube formed by winding the paper blank of Fig. 1.

DETAILED DESCRIPTION OF THE EXEMPLARY EMBODIMENTS

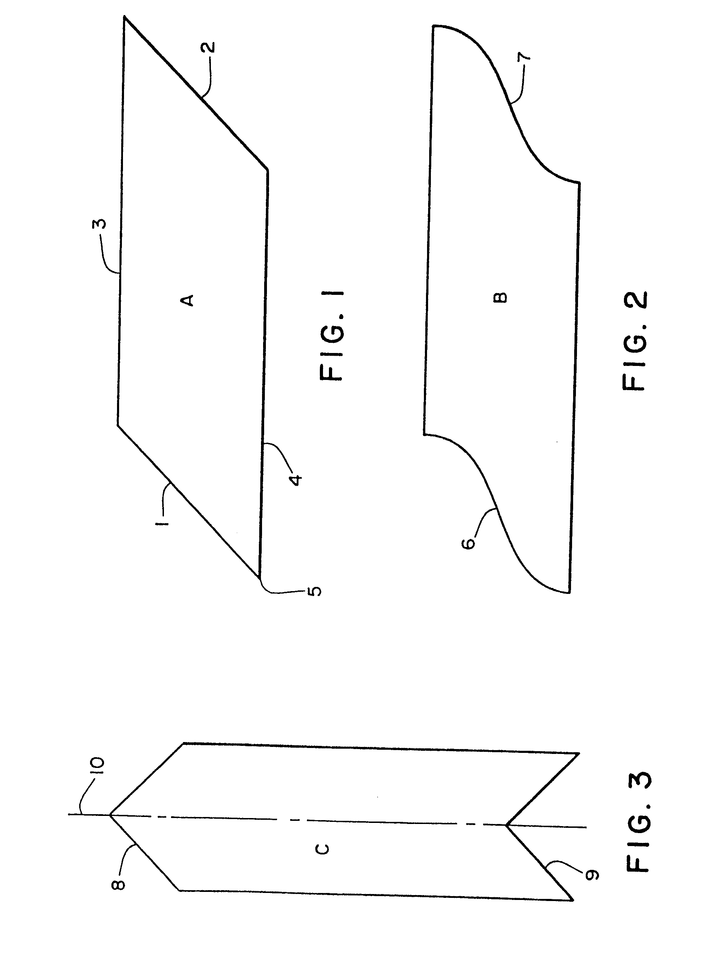

In Fig. 1 a blank of paper in the shape of a parallelogram is shown having two long edges 3 and 4 and two short edges 1 and 2. A pointed leading edge is formed by the intersection 5 of edges 1 and 4. The angle between edges 1 and 4 and edges 2 and 3 can include an angle between 5 degrees and 85 degrees, but preferably is 45 degrees. A bias cut of 45 degrees results in 41% more exposed edge on the inside and outside of the formed tube than a comparable tube formed of a rectangular blank.

The paper blank may have curved leading and trailing edges 6 and 7, respectively, as shown in Fig. 2. The reverse curve pattern of the leading and trailing edges gives even more exposed edge on the inside and outside of the finished tube.

A further exemplary embodiment of a paper blank is shown in Fig. 3, wherein leading and trailing edges 8 and 9, respectively, have a pattern of two intersecting lines to form any appropriate angle. As illustrated, each straight portion of the leading trailing edges is inclined at an angle of 45 degrees to a line 10 parallel to and midway between the long parallel edges.

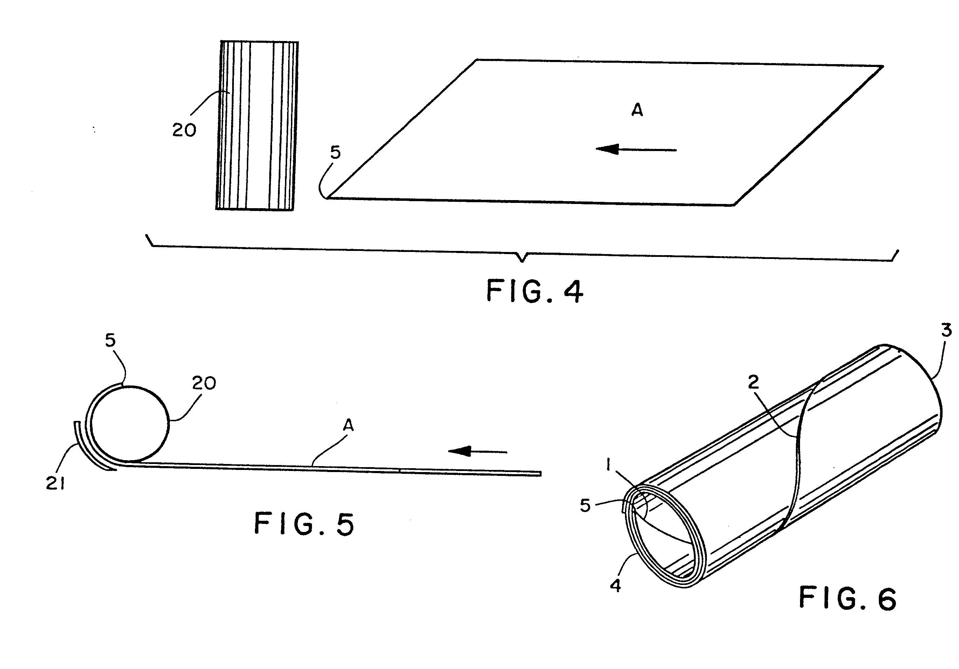

Fig. 4 illustrates the approach of paper blank A to a rotating mandrel 20. The corner 5 of the pointed leading edge initially contacts the mandrel.

8

Since a small edge initially contacts the rotating mandrel, alignment is not difficult and the starting of the winding is greatly facilitated.

Fig. 5 illustrates the beginning of the winding of blank A with corner 5 leading. A curved guide 21 for initially guiding the blank is shown schematically.

Preferably the mandrel 20 has a hollow interior into which a vacuum is drawn. Perforations are provided (not shown) for exerting a suction force on the paper to hold the leading edge and first winding securely as the blank is wound into a tube.

Fig. 6 illustrates a tube formed by a winding paper blank A on mandrel 20. As shown, edge 2 follows a spiral path on the outside of the tube giving the appearance of a spirally wound tube. Edge 1 follows a similar spiral pattern in the reverse direction on the inside of the tube. The tube formed by blank B would have edges forming similar spirals on the inside and outside of the tube, but with a sharper pitch. The tube formed by blank B would have a pair of spiraled edges on both the inside and outside of the tube. Each spiral of a pair would begin at the midpoint of the tube and proceed in a reverse direction to that of the other spiral of a pair.

In a preferred embodiment, the surface of the paper blank forming the interior of the wound coils of the tube is coated with a water soluble, heat activatable adhesive. The surface of the blank forming the exterior of the tube is coated with a thin water soluble thermoplastic such as polyvinyl alcohol, polyvinylpyrrolidone, polyethylene oxide, polyvinyl

9

acetate and hydroxypropyl cellulose to which a high percentage of a suitable clay is mixed to give a smooth slick exterior for ease of insertion and retraction by customer. The water soluble heat activatable adhesive may be of any suitable type, but is preferablly a composition including polyethyloxazoline, a compatible water dispersible adhesive and an antiblocking agent to prevent premature adhesion. Such composition is more fully described in U.S. Patent No. 4,522,967.

The number of plies of the formed tube depends on the thickness and strength of the paper used. Preferably a three ply tube formed from paper having a short-fiber, high-ground wood content and a weight of 36-50 lbs. per 1,000 square feet is used.

The width of the blanks illustrated in Figs. 1, 2 and 3 is preferably approximately 3 inches. The length of upper and lower parallel edges is approximately 8½ inches. These dimensions result in a 3 inch long tube having a three ply thickness.

After being wound on the mandrel 20, heat is applied to activate the thermoformable substrate of adhesive to securely hold the convolute windings in place. Any suitable heating means may be utilized, such as a dielectric, microwave or convection heating means. Preferably, a heated roller (not shown) is provided to rotate in conjunction with the mandrel 20 to form a nip through which the paper blank passes.

After the tube is manufactured, when used as an outer tube of the tampon applicator, an end may be suitably formed for restraining a tampon and yielding when the tampon is pushed therethrough by a plunger

10

which may be an inner telescoping tube of lesser diameter.

After use, the tubes of the tampon applicator may be disposed of by flushing down a toilet, in which case the water soluble adhesive and the extra exposed edges of the tubes of the invention cause rapid delamination and softening of the rigid tubes so that the blockage of the drain pipes from the toilet does not result.

Although particular paper blanks and a winding process have been described to illustrate exemplary embodiments of the invention, it will be appreciated that the present invention is not limited to the particular illustrations and descriptions. Accordingly, any and all modifications, alterations and equivalent arrangements for such tubes and method and apparatus for manufacturing the same falling within the scope of the following claims should be considered to be part of the invention.

11

What I Claim is:

1. A convolutely wound, disposable paper tampon tube comprising a unitary one-piece blank of paper wound into a tube adapted to confine a tampon pledget and having inner and outer face surfaces, a first edge of the paper blank forming a spiral pathway on the inner face surface of the tube and a second edge of the paper blank forming a spiral pathway on the outer face surface of the tube.

2. A convolutely wound, disposable paper tampon tube as set forth in Claim 1, wherein the tube is formed with plural windings and the first and second edges are held together by a water dispersible, heat activatable adhesive.

3. A convolutely wound, disposable paper tube as set forth in Claim 1, wherein the one-piece paper blank has two long parallel sides joined by said first and second edges, said long parallel sides forming respective ends of the tube.

4. A convolutely wound paper tube as set forth in Claim 3 wherein said first and second edges form an angle of from about 5 to about 85 degrees with respective long sides.

5. A convolutely wound paper tube as set forth in Claim 4, wherein said angle is about 45 degrees.

6. A convolutely wound paper tube as set forth in Claim 3, wherein said first and second edges are curved in the shape of a reverse curve.

12

7. A convolutely wound paper tube as set forth in Claim 3, wherein said first and second edge portions are each formed from two straight portions intersecting to form an angle thereinbetween.

8. A method of making a wound paper tampon tube comprising the steps of shaping a paper blank to have two long parallel edges joined to shorter edges, said shorter edges each having a narrow pointed portion, and winding the paper blank about an axis perpendicular to the long edges to form a convolute tube.

9. A method as set forth in Claim 8, wherein the shorter edges of the blank are each shaped to be at an angle of from about 5 to about 35 degrees with respect to a respective long edge.

10. A method as set forth in Claim 9, wherein the shorter edges are each shaped to be at an angle of 45 degrees with respect to a respective long edge.

11. A method as set forth in Claim 8, wherein said shorter edges are shaped in a curved pattern.

12. A method as set forth in Claim 11, wherein the curved pattern is a reverse curve.

13. A method as set forth in Claim 8, wherein said shorter edges are each shaped to have two sections intersecting with each other at an angle.

13

14.   A method as set forth in Claim 13, wherein said angle is 90 degrees.

15.   A method as set forth in Claim 8, and further including the step of coating the paper blank with a water soluble heat activatable adhesive prior to winding.

16.   A method as set forth in Claim 15, wherein the winding step takes place on a rotating mandrel and further including the step of heating the paper blank as it is being wound on the mandrel.

17.   A method as set forth in Claim 8, wherein the step of winding takes place on a rotating mandrel and further including the step of holding the blank on the mandrel by suction by providing a hollow mandrel with perforations and a vacuum source for evacuating air from the interior of the mandrel.

18.   A method of forming a convolutely wound paper tampon tube comprising the steps of forming a narrow leading edge on a paper blank, feeding the paper blank towards the rotating mandrel with the leading edge foremost and winding the paper blank on the mandrel to form a plural layered paper tampon tube.

19.   A method as set forth in Claim 18 and further including the step of forming a trailing edge

14

on the paper blank to have a pattern identical to said leading edge.

20. A method as set forth in Claim 18 and further including the step of coating a surface of the paper blank with a water soluble heat activatable adhesive.

FIG. 1

FIG. 2

FIG. 3

FIG. 4

FIG. 5

FIG. 6

0220091

**0220091**

Application number

## EUROPEAN SEARCH REPORT

European Patent Office

EP 86 40 2022

### DOCUMENTS CONSIDERED TO BE RELEVANT

| Category | Citation of document with indication, where appropriate, of relevant passages | Relevant to claim | |
|---|---|---|---|
| D,Y | US-A-3 764 438  (VOSS et al.)<br>* figure 2 *<br>--- | 1 | **CLASSIFICATION OF THE APPLICATION (Int Cl 4)**<br><br>A 61 F   13/20<br>B 31 C    1/00 |
| Y | FR-A- 939 875  (STE ALSACIENNE D'EXPLOSIFS ET D'APPLICATIONS CHIMIQUES)<br>* entire document * | 1 | |
| A | | 3-5,8, 17-19 | |
| A | DE-C-1 089 255  (REIS)<br>* figures 3, 4 *<br>--- | 1,7 | |
| A | US-A-3 105 421  (PETRI)<br><br>* figures 3, 4 *<br>--- | 1,3-5, 7 | **TECHNICAL FIELDS SEARCHED (Int Cl 4)** |
| D,A | US-A-4 522 967  (SHELDON et al.)<br>* claim 1; figure *<br>----- | 1,2 | A 61 F   13/00<br>B 31 C    1/00<br>B 31 C   13/00 |

The present search report has been drawn up for all claims

| Place of search | Date of completion of the search | Examiner |
|---|---|---|
| BERLIN | 02-12-1986 | KANAL P K |